# EUROPÄISCHE PATENTSCHRIFT

(11) **EP 1 062 940 B1**
(45) Veröffentlichungstag und Bekanntmachung des Hinweises auf die Patenterteilung: **25.11.2009**
(21) Anmeldenummer: 99901954.0
(22) Anmeldetag: 08.02.1999
(51) Int. Cl.: A61K 8/41, A61K 8/49, A61Q 5/06, A61Q 5/10

(54) **DAUERWELLZUSAMMENSETZUNG MIT FÄRBENDEM EFFEKT UND VERFAHREN ZUR HAARFÄRBUNG UNTER VERWENDUNG DERSELBEN**
PERMANENT WAVE COMPOSITION HAVING DYEING EFFECT AND METHOD FOR DYEING HAIR USING THE SAME
COMPOSITION D'AGENT POUR PERMANENTE AYANT UN EFFET COLORANT ET SON PROCEDE DE COLORATION DES CHEVEUX

(30) Priorität: 10.02.1998 JP 2879798
(43) Veröffentlichungstag der Anmeldung: 27.12.2000
(73) Patentinhaber: SCHWARZKOPF & HENKEL K.K., Shinagawa-ku Tokyo (JP)
(72) Erfinder: TSUJINO, Yoshio Yamahatsu Sangyo Kaisha, Ltd., Osaka-shi Osaka 557-0055 (JP); KAMISHITA, Takako Yamahatsu Sangyo Kaisha, Ltd., Osaka-shi Osaka 557-0055 (JP)
(74) Vertreter: Stevermann, Birgit
(86) Internationale Anmeldenummer: PCT/JP1999/000523
(87) Internationale Veröffentlichungsnummer: WO 1999/040895

(56) Entgegenhaltungen:
- EP-A- 0 850 637
- WO-A-97/39727
- FR-A- 2 741 798
- GB-A- 1 491 930
- JP-A- 8 507 545
- JP-A- 9 151 121
- JP-A- 55 022 638
- US-A- 4 025 301

## Beschreibung

### Technische Anwendungsgebiete

Die vorliegende Erfindung betrifft eine Dauerwellmittelformulierung mit einer haarfärbenden Wirkung und ein Verfahren zum Haarfärben unter Verwendung derselben.

### Technische Hintergründe

Im allgemeinen besteht ein Dauerwellmittel aus einem ersten Mittel (Reduktionsmittel), das Thioglykolsäure usw. als Hauptbestandteil enthält, und einer zweiten Teilkomposition (oxidierendes Fixiermittel), das Wasserstoffperoxid, Kaliumbromat oder Natriumbromat als Hauptbestandteil enthält. Bisher sind aber keine Dauerwellmitteltypen, die zusätzlich mit einer haarfärbenden Wirkung versehen sind, anzutreffen.

In dieser Situation liegt die erste Aufgabe der Erfindung darin, ein Dauerwellmittel, das mit einer haarfärbenden Wirkung versehen ist, anzubieten, das hohe Stabilität, gutes Färbevermögen und hohe Echtheit aufweist. Die zweite Aufgabe der Erfindung ist es, ein Verfahren zum Färben von Haaren anzubieten, das dadurch gekennzeichnet ist, daß Haare, die einer Dauerwellbehandlung unterzogen worden sind, mit der vorgenannten Zusammensetzung gefärbt werden.

### Offenbarung der Erfindung

Um ein Dauerwellmittel mit einer haarfärbenden Wirkung zu erhalten, haben die Erfinder der zweiten Teilkomposition des Dauerwellmittels Farbstoffe, darunter saure Farbstoffe und Teerfarbstoffe, die üblicherweise als Haarfarbstoffe verwendet werden, zugesetzt und die Stabilität, das Färbevermögen und die Echtheit gegen Licht bzw. Hydrolyse (nachstehend einfach Echtheit genannt) überprüft. Dabei haben sie gefunden, daß in den letzten Jahren als geeignete Farbstoffe für das Haarfärben vorgeschlagene kationische Farbstoffe (EP 714954A; WO95/01772 und WO 95/15144[JP-A-8-507545]) in Hinsicht auf Stabilität, Färbevermögen und Echtheit jeweils die höchsten Werte aufweisen. Weiterhin hat es sich ergeben, daß beim Färben der dauerwellbehandelten Haare mit diesen kationischen Farbstoffen ein hohes Färbevermögen ohne Beeinflussung des Dauerwelleffektes erreicht wird.

So wurde die Erfindung auf Basis der neuartigen Erkenntnisse von dem Erfinder vollendet. Erfindungsgemäß wird gemäß der Ausführungsform der Erfindung eine zweite Teilkomposition des Dauerwellmittels, die einen kationischen Farbstoff enthält, der ein quartäres Stickstoffatom, dessen freies Elektronenpaar delokalisiert sein kann, und eine -X=N-Bindung aufweist, wobei X für ein Stickstoffatom oder die Gruppe -CH- steht, zur Verfügung gestellt.

So kann in der Ausführungsform der Erfindung eine zweite Teilkomposition des Dauerwellmittels mit hohem Färbevermögen und guten Echtheiten zur Verfügung gestellt werden.

Im folgenden sei die Erfindung gemäß der Ausführungsform näher erläutert:

### [Ausführungsform]

Bei der zweiten Teilkomposition des Dauerwellmittels gemäß der Ausführungsform der Erfindung handelt es sich um die Formulierung, die durch Mischung bzw. Kombination der bekannten zweiten Teilkomposition des Dauerwellmittels mit den o. g. kationischen Farbstoffen erhalten wird.

Als konkretes Beispiel für die in der ersten Ausführungsform verwendeten kationischen Farbstoffe sind die der Formel(I) anzuführen :

[A-Z=N-B]⁺X⁻ (I)

worin
Z Stickstoff oder -CH- bedeutet,
A und B einen aromatischen Benzolring oder Heterozyklus, die gewünschtenfalls mit einem oder mehreren Halogenatomen oder einer oder mehreren NR₁R₂- oder OR₁-Gruppen substituiert sein können, darstellt, wobei
R₁ und R₂ gleich oder verschieden sein können und unabhängig voneinander bedeuten Wasserstoff, C₁₋₈Alkyl, C₁₋₄Hydroxyalkyl oder Phenyl, und
X⁻ ein Anion ist.

Diese Farbstoffe sind in EP 714954A, WO95/01772 und WO 95/15144 (JP 8/507545) ausführlich beschrieben.

Als bevorzugte Anionen werden u. a. Chlorid und Methylsulfat angeführt. Typische Beispiele für diese kationischen Farbstoffe sind:
4-Aminophenylazo-2-hydroxy-7-trimethylammoniumnaphthalinchlorid mit der Formel (1), 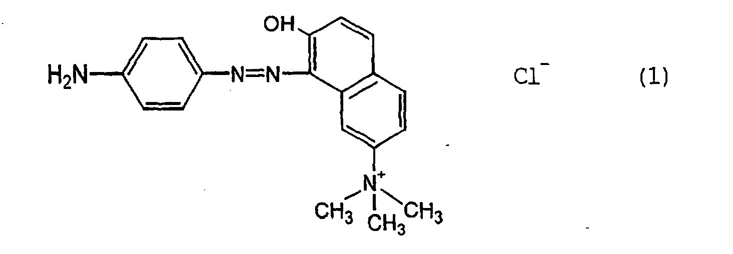
2-Methoxyphenylazo-2-hydroxy-7-trimethylammoniumnaphthalmchlorid mit der Formel (2), 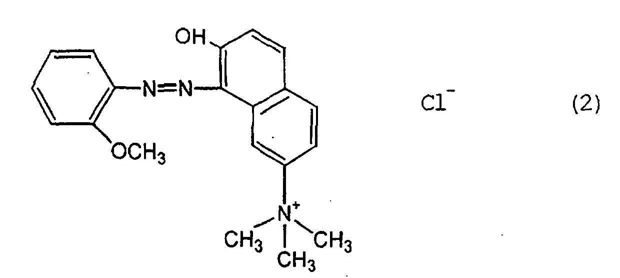
4-Amino-3-nitrophenylazo-2-hydroxy-7-trimethylammoniumnaphthalinchlorid mit der Formel (3), 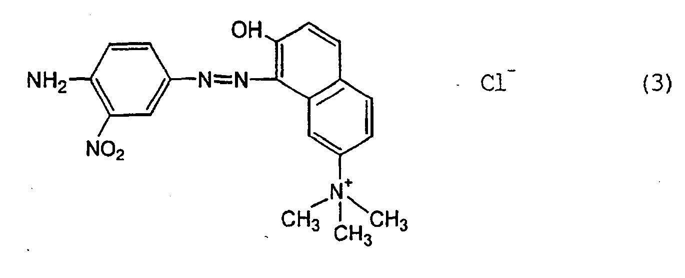
3-Trimethylammoniumphenylazo-4N-phenyl-2-methyl-5-hydroxypyrazolchlorid mit der Formel (4), 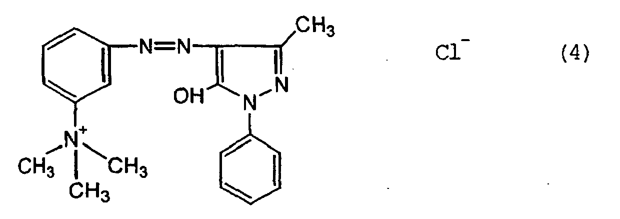
(1-Methyl-1-phenyl)-2-(1-methin-4N-methylpyridinium) hydrazinchlorid mit der Formel (5), 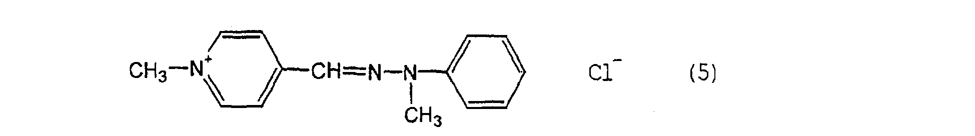
(1-Methyl-1-para-methoxyphenyl)-2-(1-methin-4N-methylpyridinium)hydrazin-chlorid mit der Formel (6), 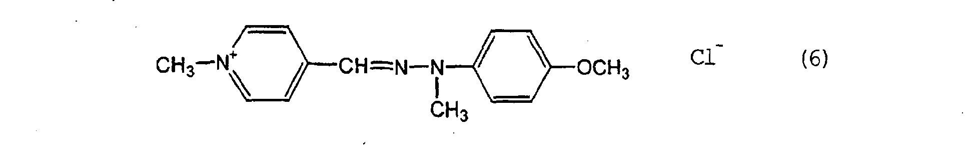
(1-Methyl-1-para-methoxyphenyl)-2-(1-methin-4N-methylpyridinium)hydrazinmethylsulfat mit der Formel (7), 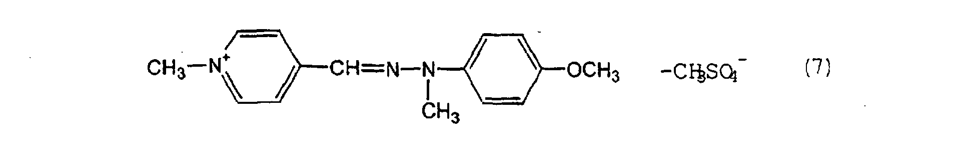
4-Dimethylaminophenylazo-2N-methyl-5N-methylimidazoliumchlorid mit der Formel (8), 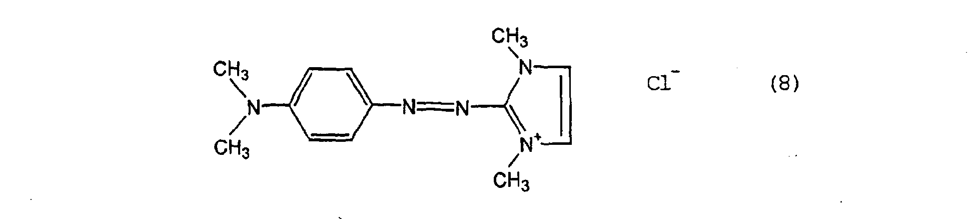
4-Dimethylaminophenylazo-2N-methyl-3N-methylpyrazoliumchlorid mit der Formel (9), 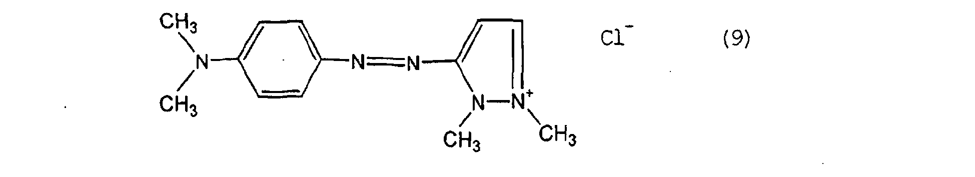
4-Methylaminophenylazo-2N-methyl-5N-methylimidazoliumchlorid mit der Formel (10), 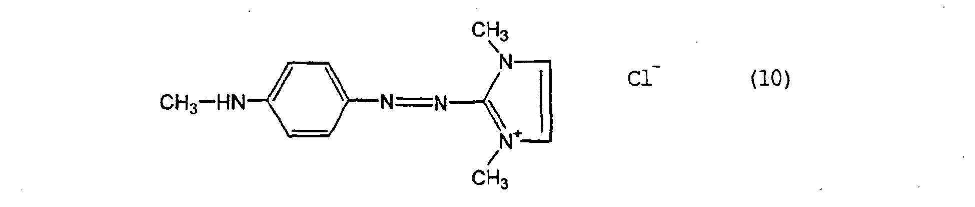
4-Aminophenylazo-2N-methyl-5N-methylimidazoliumchlorid mit der Formel (11), 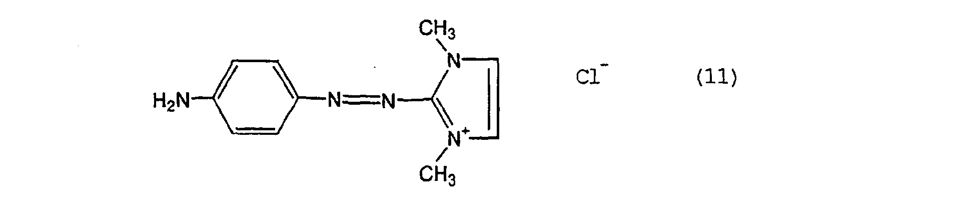
4-Dimethylaminophenylazo-4N-methylpyridiniumchlorid mit der Formel (12), 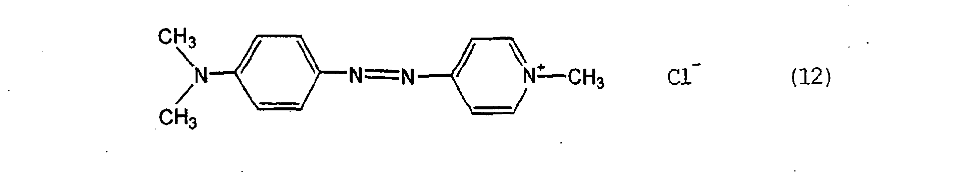
4-Dimethylaminophenylazo-4N-hydroxypyridiniumchlorid mit der Formel (13).

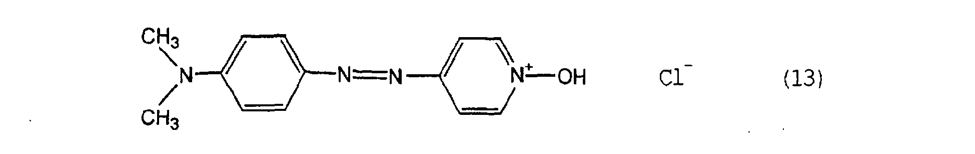

Diese kationischen Farbstoffe angemessen sowohl allein als auch in Kombination von 2 oder mehr Vertretern verwendet werden. Die Farbstoffeinsatzmenge ist je nach den gewünschten Farbtönen angemessen zu wählen, beträgt aber üblicherweise 0,001- 3 Gew.-%, bezogen auf das Gesamtgewicht der zweiten Teilkomposition.

In der ersten Ausführungsform der Erfindung kann die zweite Teilkomposition des Dauerwellmittels nach üblichem Verfahren formuliert werden, wobei die gewünschten Bestandteile, die o. g. kationischen Farbstoffe und das Oxidationsmittel, wie Wasserstoffperoxid, Kaliumbromat, Natriumbromat, Natriumperborat oder deren Mischungen, die für die zweite Teilkomposition des Dauerwellmittels unerläßlich sind, gemischt werden. Dazu werden ggf. die bekannten üblichen geeigneten weiteren Inhaltsstoffe, wie Löse-, Penetrationshilfs-, Netz-, Haarkräftigungs-, Emulgiermittel, Parfüme usw. zugesetzt. Die Farbstoffe und die Oxidationsfixiermittel können auch als getrennte Formulierungen in Form eines Zwei-Komponenten-Mittels konfektioniert werden, die beide erst beim Verwenden miteinander gemischt werden. Die zweite Teilkomposition des Dauerwellmittels in der Ausführungsform der Erfindung ist in gleicher Weise wie zweite Teilkompositionen üblicher Dauerwellmittel anwendbar. Wenn die Farbstoffe und die Oxydationsfixiermittel voneinander getrennt formuliert sind, können sie beim Verwenden miteinander gemischt werden.

### Beste Ausführungsform für die Durchführung der Erfindung

Im folgenden soll die Erfindung an Prüfungs- und Ausführungsbeispielen näher erläutert werden; allerdings beschränkt sie sich nicht auf diese Beispiele. Im nachstehenden bedeutet die %-Angabe Gew.-%, wenn nicht anders vermerkt.

### Prüfungsbeispiel 1

Stabilität der zweiten Teilkomposition des Dauerwellmittels mit direktziehenden Farbstoffen im Zeitverlauf.

Die sauren Farbstoffe, Teerfarbstoffe und kationischen Farbstoffe als direktziehende Farbstoffe wurden so eingewogen, daß ihre Aktivanteile 0,2 Gew.-%, bezogen auf das Gesamtgewicht der jeweiligen zweiten Teilkomposition des Dauerwellmittels entsprechen, dann wurden sie dem Oxidationsfixiermittel, dessen Bestandteile in Tabelle 1 gezeigt sind, zugesetzt und umgerührt.

Als saure Farbstoffe wurden Black 401, Violet 401 und Orange 205 verwendet, als Teerfarbstoffe HC Blue 2 und HC Yellow 2 (Fa. JAMES ROBINSON), als kationische Farbstoffe (A) (1 -Methyl-1 -para-methoxy-phenyl)-2-(1-methin-4N-Methylpyridinium) hydrazinchlorid,(B) 4-Dimethylaminophenylazo-2N-methyl-5N-methylimidazoliumchlorid und (C) 4-Aminophenylazo-2N-methyl-5N-methylimidazoliumchlorid (Fa. Ciba Specialty Chemicals).

**Tabelle 1**

| Inhaltsstoffe | Einsatzmenge (%) |
|---|---|
| Natriumbromat | 8.50 |
| Trinatriumphosphat | 0.27 |
| Phosphorsäure | 0.09 |
| Gereinigtes Wasser | Ausgleich |

Nach etwa 10-minütigem Rühren bei Raumtemperatur wurde die zweite Teilkomposition des Dauerwellmittels, die den Farbstoff enthielt, durch Filterpapier (TOYO ADVANTEC No.2, Durchmesser: 125mm) abfiltiert, um die Probe zu erhalten. Die Probe wurde bei 45°C gelagert und die Stabilität der Probe bezüglich Löslichkeit der Farbstoffe, dem pH-Wert und der Absorptionsspektren im sichtbaren Bereich bewertet.

Die Bewertung der Farbstofflöslichkeit wurde nach folgendem Kriterium durchgeführt:
O : Keine Veränderung im Vergleich zu dem Meßwert vor zwei Wochen
X : Ausfällen der Farbstoffe

Der pH-Wert wurde mittels eines pH-Meters von Fa. HORIBA gemessen. Zur Bewertung der Absorptionsspektren im sichtbaren Bereich wurde die Extinktion bei der Wellenlänge des Absorptionsmaximums mit HITACHI U-3210 (ausgerüstet mit einer Quarzküvette von 10mm Strahlenganglänge) gemessen und das Verhältnis der Restmenge an den gelösten Farbstoffen nach 4 Wochen gegenüber dem Anfangswert (Bezug: 100) errechnet.

Die Ergebnisse sind in Tabelle 2 angegeben :

**Tabelle 2 :**

| | Black 401 | Violet 401 | Orange 205 | HC BLUE | HC YELL- | Kationischer Farbstoff Typ | | |
|---|---|---|---|---|---|---|---|---|
| | | | | 2 | OW2 | (A) | (B) | (C) |
| Farbstofflöslichkeit | | | | | | | | |
| (45°C) | | | | | | | | |
| nach 2 Wochen | ○ | ○ | ○ | ○ | ○ | ○ | ○ | ○ |
| nach 4 Wochen | X | X | X | ○ | ○ | ○ | ○ | ○ |
| pH-Wert am Anfang | 6.43 | 6.54 | 6.44 | 6.43 | 6.43 | 6.43 | 6.42 | 6.41 |
| (45°C) nach 2 Wochen | 6.44 | 6.52 | 6.43 | 6.44 | 6.42 | 6.42 | 6.41 | 6.40 |
| nach 4 Wochen | 6.45 | 6.54 | 6.45 | 6.15 | 6.44 | 6.42 | 6.43 | 6.41 |
| Restfarbstoffmenge nach 4 Wochen (bei 45°C) | 87.0 | 92.2 | 98.7 | 0.00 | 97.6 | 99.6 | 102.0 | 98.0 |

Aus Tabelle 2 ergibt sich, daß bei der Verwendung von Farbstoffen als Dauerwellmittelzusätze die kationischen Farbstoffe und der Teerfarbstoff HC Yellow 2 im Zeitverlauf stabil sind.

### Prüfungsbeispiel 2

Prüfung der zweiten Teilkomposition des Dauerwellmittels mit Farbstoffen auf ihr Färbevermögen und ihre Echtheiten.

Ca. 2g der ersten Teilkomposition des Dauerwellmittels wurden auf ca. 2g Ziegenhaare aufgetragen und im Trockenschrank 15 Minuten lang bei 30°C belassen. Dann wurden ca. 2g abfiltrierte zweite Teilkomposition des Dauerwellmittels mit Farbstoffen aus dem ersten Prüfungsbeispiel auf die Haare aufgetragen.

Die so gefärbten Ziegenhaare wurden schampuniert, mit einer konditionierenden Spülung behandelt und mit Wasser gespült. Nach dem Trocknen wurde die Färbung der Haare bewertet.

Darüber hinaus wurden die Haare, die bei der oben beschriebenen Prüfung angefärbt waren, nach dem Färben 24 Stunden lang belassen; anschließend wurden sie in 300ml einer wäßrigen Lösung von 0,03% Polyoxyäthylen (2EO) alkyl *(C₁₂, ₁₃)* ethernatriumsulfat 10 Minuten lang bei 80°C getaucht, danach mit warmem Wasser gründlich gespült und getrocknet. Nach dieser Behandlung wurde die Echtheit der Haarfärbung bewertet. Dabei basiert die Bewertung auf folgendem Kriterium :
• : intensive Färbung, das ursprüngliche Grau der Ziegenhaare ist nicht mehr erkennbar
○ : so tiefe Färbung, daß das ursprüngliche Grau der Ziegenhaare mehr oder weniger nicht mehr auffällt
△ : die Haare sind gefärbt, aber das ursprüngliche Grau der Ziegenhaare fällt noch auf.
× : nur die ursprünglich graue Farbe der Ziegenhaare ist erkennbar

Die Ergebnisse sind in Tabelle 3 zusammengestellt:

**Tabelle 3 :**

| | Anfärbung | Echtheit |
|---|---|---|
| Black 401 | ○ | × |
| Violet 401 | × | × |
| Orange 205 | △ | × |
| HC Blue 2 | ○ | × |
| HC Yellow 2 | ○ | × |
| Kationischer Farbstoff (A) | • | • |
| Kationischer Farbstoff (B) | • | • |
| Kationischer Farbstoff (C) | • | • |

Wie in Tabelle 3 gezeigt, wiesen die kationischen Farbstoffe in Hinsicht auf das Färbevermögen und die Echtheit die höchsten Werte auf.

### Prüfungsbeispiel 3

Messung des Wellwertes und des Wellerhaltungsgrads beim Haarfärben mit der zweiten Teilkomposition des Dauerwellmittels enthaltend Farbstoffe und bei Färben der im voraus dauerwellbehandelten Haare mit wäßriger Lösung des kationischen Farbstoffs.

Der Wellwert wurde nach der Kirby-Methode mit Hilfe von Menschenhaaren bewertet. Beim Haarfärben mit der zweiten Teilkomposition des Dauerwellmittels enthaltend Farbstoffe wurde eine Probe nacheinander in die 1. und die 2. Teilkomposition (je 50g) getaucht, wobei jeweils die Behandlungstemperatur 30°C und die Behandlungszeit 15 Minuten betrug. Bei dieser Prüfung wurde die Probe nach der Behandlung mit der zweiten Teilkomposition des Dauerwellmittels enthaltend 0,05% kationischen Farbstoff (B) gespült und vom Gerät abgenommen. Dann wurde der Wellwert gemessen.

Beim Färben der Haare nach der Dauerwellbehandlung wurde die Probe mit der ersten Teilkomposition des Dauerwellmittels und der zweiten ohne Farbstoffe auf gleiche Weise wie oben erwähnt behandelt, nach der Behandlung mit der zweiten Teilkomposition gründlich gespült und in die 0,05%ige wäßrige Lösung des kationischen Farbstoffs (B), die auf pH 9 eingestellt war, getaucht, wobei sie 30 Minuten bei 30°C behandelt wurde. Danach wurde sie gründlich gespült und vom Gerät abgenommen. Dann wurde der Wellwert gemessen. Bei der Prüfung des Wellerhaltungsgrads wurde die Haarprobe nach dem Messen des Wellenwertes der Belastungsprüfung unterzogen, bei der sie in 20% Natriumlaurylsulfat-Lösung 20 Minuten bei 60°C erwärmt wurde, und danach wurde der Wellwert, wie oben beschrieben, gemessen. Daraufhin wurde das Verhältnis der Wellwerte nach und vor der Belastungsprüfung berechnet und verglichen.

Die Kontrollprobe, die mit der ersten Teilkomposition des Dauerwellmittels und der zweiten Teilkomposition des Dauerwellmittels ohne Farbstoffe behandelt wurde, wurde ebenfalls dem Belastungstest unterzogen. Der Wellwert wurde vor und nach dem Test gemessen, um den Wellerhaltungsgrad zu ermitteln.

Die Ergebnisse sind in Tabelle 4 gezeigt :

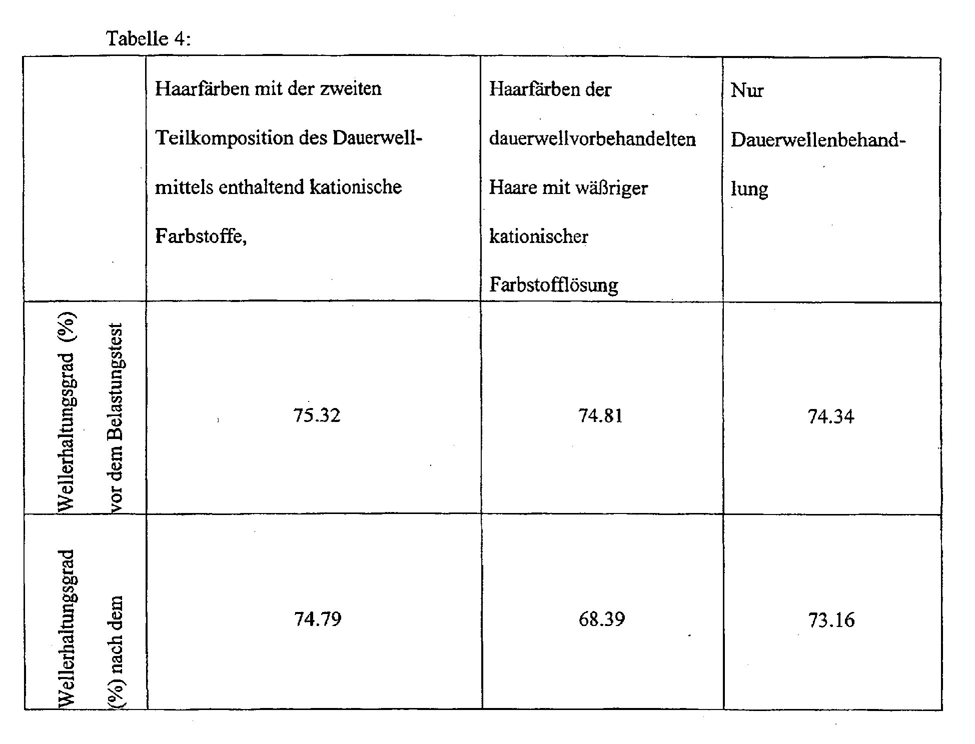

Wie in Tabelle 4 gezeigt, beeinflussen die kationischen Farbstoffe den Dauerwelleffekt nicht.

### Prüfungsbeispiel 4

Färbevermögen der zweiten Teilkomposition des Dauerwellmittels enthaltend kationische Farbstoffe und Färbevermögen der kationischen Farbstofflösung für dauerwellvorbehandelte Haare.

Ca. 2g Ziegenhaar, auf das die erste Teilkomposition des Dauerwellmittels und dann 2g der zweiten Teilkomposition des Dauerwellmittels enthaltend 0,05% kationischen Farbstoff(B) aufgetragen wurden, wurden im Trockenschrank 15 Minuten lang bei 30°C belassen. Die Probe wurde nach der Behandlung mit der zweiten Teilkomposition schampuniert, mit einer konditionierenden Spülung behandelt, gründlich gespült und getrocknet. Danach wurde das Färbevermögen bewertet.

Eine andere Probe, auf die die erste Teilkomposition und 2g der zweite Teilkomposition ohne Farbstoffe aufgetragen wurden, wurde unter gleichen Bedingungen wie im o. a. Beispiel dauerwellbehandelt. Nach dem Behandeln mit der zweiten Teilkomposition wurde die Probe gründlich gespült. Diese Probe und eine nicht behandelte Haarprobe, auf die jeweils 2g der 0,05 %igen wäßrigen Lösung des kationischen Farbstoffs (B), die auf pH 9 eingestellt war, aufgetragen wurden, wurden 30 Minuten lang bei 30°C belassen. Anschließend wurden beide Proben schampuniert, mit einer konditionierenden Spülung behandelt, gründlich gespült und getrocknet. Danach wurde das Färbevermögen bewertet. Dabei basierte die Bewertung auf der Helligkeitsstandardfarbskala nach JIS. Je kleiner dieser Wert wird, um so höher ist das Färbevermögen.

Die Ergebnisse sind in Tabelle 5 gezeigt :

**Tabelle 5:**

| Bedingungen für das Haarfärben | Haarfärben mit der zweiten Dauerwellmittelkomponente enthaltend kationischen Farbstoff, | Färben der dauerwellvorbehandelten Haare mit wäßriger Lösung des kationischen Farbstoffes, | Färben der nicht behandelten Haare mit wäßriger Lösung des kationischen Farbstoffs. |
|---|---|---|---|
| Farbausbeute | 4 | 3.5 | 5 |

Wie Tabelle 5 zeigt, wird die Farbausbeute durch die Dauerwellbehandlung erhöht.

### Prüfungsbeispiel 5

Einflüsse des pH-Werts der kationischen Farbstofflösung auf das Färbevermögen. Ca. 2g Ziegenhaar, auf das 4g der 0,05%igen wäßrigen Lösung des kationischen Farbstoffes (C), die auf pH-Werte von 3, 5, 7, 9 oder 11 eingestellt war, aufgetragen wurden, wurden im Trockenschrank 30 Minuten bei 30°C verweilen gelassen, dann schampuniert, mit einer konditionierenden Spülung behandelt, gründlich mit Wasser gespült und getrocknet. Danach wurde das Färbevermögen wie bei Prüfungsbeispiel 4 bewertet.

Die Ergebnisse sind in Tabelle 6 gezeigt :

**Tabelle 6**

| | | | | | |
|---|---|---|---|---|---|
| pH | 3 | 5 | 7 | 9 | 11 |
| Farbausbeute | 6.5 | 6.0 | 5.7 | 5.3 | 5.0 |

Wie Tabelle 6 zeigt, steigt das Färbevermögen mit der Erhöhung des pH-Werts der Lösung des kationischen Farbstoffes. Aber die Lösung mit einem pH-Wert ≥10 ist wegen ihres starken Reizes für den menschlichen Körper ungeeignet, also sollte die Lösung auf den pH-Bereich von 5≤pH<10, vorzugsweise auf pH 9, eingestellt werden. Nun ist die zweite Dauerwellmittelkomponente im allgemeinen auf pH 4-10,5, vorwiegend auf pH 6-8, eingestellt. Folglich ist ein hohes Färbevermögen zu erwarten, auch wenn kationische Farbstoffe im voraus mit der zweiten Teilkomposition des Dauerwellmittels gemischt sind. Diese Prüfung, in der auch nicht dauerwellbehandelte Ziegenhaare gefärbt wurden, läßt vermuten, daß zwar beim Färben der dauerwellbehandelten Haare höhere Farbausbeuten erzielen werden als beim Färben der nicht dauerwellbehandelten Haare, aber in Hinsicht auf die Abhängigkeit des Färbevermögens vom pH-Wert beide Färbungen ein gleiches Verhalten zeigen.

### Prüfungsbeispiel 6

Einflüsse des Wassergehalts in der wäßrigen Farbstofflösung auf das Färbevermögen kationischer Farbstoffe.

0,05%ige wäßrige Lösungen des kationischen Farbstoffs (C), in die jeweils 0%,10%, 20%, 30%, 40% bzw. 50% Propylenglykol, bezogen auf das Gewicht der Farbstofflösung, gemischt sind, wurden jeweils auf pH 9 eingestellt. Ca. 2g Ziegenhaar, auf das jeweils 4g jeder Farbstofflösung aufgetragen wurden, wurden im Trockenschrank 30 Minuten bei 30°C belassen, dann schampuniert, mit einer konditionierenden Spülung behandelt und gründlich mit Wasser gespült. Danach wurde die Farbausbeute wie bei Prüfungsbeispiel 4 bewertet.

Die Ergebnisse sind in Tabelle 7 gezeigt:

**Tabelle 7**

| Propylenglykol (in Gew.-%) | 0 | 10 | 20 | 30 | 40 | 50 |
|---|---|---|---|---|---|---|
| Farbausbeute | 6.5 | 6.8 | 7.0 | 7.7 | 8.5 | 9.0 |

Wie in Tabelle 7 gezeigt, nimmt das Färbevermögen bei Propylenglykolmengen ≥ 30%, besonders von 40 und 50% erheblich ab; deshalb sollte die Wassermenge mindestens 70%, vorzugsweise 80% und darüber, betragen.

### Ausführungsbeispiel 1

Zweite Teilkomposition des Dauerwellmittels mit Wasserstoffperoxid.

Die zweite Teilkomposition mit folgender Zusammensetzung wurde nach üblichem Verfahren hergestellt :

| Inhaltsstoffe | % |
|---|---|
| (1-Methyl-1-phenyl)-2-(1-methin-4N-methylpyridinium) | |
| hydrazinmethylsulfat | 0,2 |
| Wäßriges Wasserstoffperoxid 35%ig | 4,3 |
| Cetanol | 0,5 |
| hydriertes Lanolin | 0,35 |
| Acetanilid | 0,02 |
| Natriumpyrophosphat | 0,025 |
| Phosphorsäure, gereinigtes Wasser | ad 100 |

(mit Phosphorsäure auf pH 6,5 eingestellt)

Die erste Teilkomposition besteht aus folgenden Bestandteilen :

| Inhaltsstoffe | % |
|---|---|
| Ammoniumthioglykolatlösung | 13,6 |
| (Konzentration : 50% Thioglykolsäure) | |
| Ammoniumbicarbonat | 3,5 |
| Dinatriumedetat | 0,1 |
| Monoethanolamin, gereinigtes Wasser | ad 100 |

(mit Monoethanolamin auf pH 9,0 eingestellt)

Nach üblicher Weise wurde graues Haar, dessen Spitzen in Papier eingewickelt waren, um einen Kunststoffstab von 1,5 cm Durchmesser gewickelt, zur Dauerwellbehandlung und Färbung in die o. g. erste Teilkomposition eingetaucht und 15 Minuten bei 30°C darin belassen, danach mit fließendem Wasser 1 Minute gespült, anschließend in die zweite Teilkomposition eingetaucht und 15 Minuten bei 30°C darin belassen, danach mit Wasser gespült und schampuniert.

Hierdurch wurde das graue Haar von der Wurzel bis zur Spitze gleichmäßig in Wellen gelegt und gelb gefärbt.

### Ausführungsbeispiel 2

### Zweite Teilkomposition des Dauerwellmittels mit Kaliumbromat

Die zweite Teilkomposition mit folgender Zusammensetzung wurde nach üblichem Verfahren hergestellt :

| Inhaltsstoffe | % |
|---|---|
| 4-Dimethylaminophenylazo-2N-methyl-5N-methylimidazoliumchlorid | 0,2 |
| Kaliumbromat | 10,2 |
| Lauryldimethylessigsäurebetain | 1,0 |
| Cetyltrimethylammoniumchlorid | 0,6 |
| Natriumbenzoat | 0,3 |
| Salicylsäure | 0,05 |
| Trinatriumphosphat | 0,27 |
| Phosphorsäure, gereinigtes Wasser | ad 100 |

(mit Phosphorsäure auf pH 6,5 eingestellt)

Die erste Teilkomposition besteht aus folgenden Bestandteilen :

| Inhaltsstoffe | % |
|---|---|
| L - Cystein-hydrochlorid | 7,0 |
| Cetanol | 0,5 |
| Oleylalkohol | 0,5 |
| Polyoxyethylencetylether ( 10 EO ) | 1,0 |
| Polyoxyethylencetylether ( 15 EO ) | 1,0 |
| Dinatriumedetat | 0,1 |
| Monoethanolamin, gereinigtes Wasser | ad 100 |

(mit Monoethanolamin auf pH 9,0 eingestellt)

Wie im Beispiel 1 wurde graues Haar dem Dauerwellbehandlungs- und Färbevorgang mit der o. g. ersten bzw. der zweiten Teilkomposition unterzogen. Hierdurch wurde das graue Haar von der Wurzel bis zur Spitze gleichmäßig in Wellen gelegt und rot gefärbt.

### Ausführungsbeispiel 3

Zweite Dauerwellmittelkomponentenformulierung mit Enzym.

Die zweite Teilkomposition mit folgender Zusammensetzung wurde nach üblichem Verfahren hergestellt. Dabei wurde Uricase unmittelbar vor der Anwendung der zweiten Teilkomposition zugesetzt.

| Inhaltsstoffe | % |
|---|---|
| 4-Aminophenylazo-2N-methyl-5N- | 0,2 |
| methylimidazoliumchlorid | |
| Uricase (20 U/ mg ) | 1,0 |
| Harnsäure | 1,0 |
| Glycerin | 3,0 |
| gereinigtes Wasser | ad 100 |

Die erste Teilkomposition besteht aus folgenden Bestandteilen :

| Inhaltsstoffe | % |
|---|---|
| Ammoniumthioglykolatlösung | 13,0 |
| (Konzentration: 50% Thioglykolsäure) | |
| Polyoxyethylencetylether ( 10 EO ) | 1,0 |
| Polyoxyethylencetylether ( 20 EO ) | 1,0 |
| Natriumlaurylsulfat | 0,5 |
| Kollagenhydrolysat-Lösung | 0,4 |
| Dinatriumedetat | 0,1 |
| Ammoniakwasser, gereinigtes Wasser | ad 100 |

(mit Ammoniakwasser auf pH 9,0 eingestellt)

Wie im Beispiel 1 wurde graues Haar dem Dauerwellbehandlungs- und Färbevorgang unterzogen. Hierdurch wurde das graue Haar von der Wurzel bis zur Spitze gleichmäßig in Wellen gelegt und braun gefärbt.

### Ausführungsbeispiel 4

Zweite Teilkomposition des Dauerwellmittels mit Natriumbromat.

Nach üblichem Verfahren wurde die zweite Teilkomposition des Dauerwellmittels als eine 2K-Zusammensetzung, bestehend aus einem Oxidationsfixiermittel und einer Farbstofflösung, gemäß folgenden Rezepturen zubereitet.

### Oxidationsfixiermittel :

| Inhaltsstoffe | % |
|---|---|
| Natriumbromat | 17,0 |
| Lauryldimethylessigsäurebetain | 1,5 |
| Cetyltrimethylammoniumchlorid | 1,0 |
| Natriumbenzoat | 0,6 |
| Salicylsäure | 0,1 |
| Trinatriumphosphat | 0,54 |
| Phosphorsäure, gereinigtes Wasser | ad 100 |

(mit Phosphorsäure auf pH 6,5 eingestellt)

### Farbstofflösung :

| Inhaltsstoffe | % |
|---|---|
| 4-(4-Aminophenylamino)phenylazo-2N-methyl-5N-methylimidazoliumchlorid | 0,4 |
| Monoethanolamin, gereinigtes Wasser | ad 100 |

(mit Monoethanolamin auf pH 8,0 eingestellt).

Als erste Teilkomposition wurde eine solche wie im Beispiel 3 verwendet.

Graues Haar wurde auf gleiche Weise wie im Beispiel 1 der Dauerwell- und Färbebehandlung unterzogen, außer daß die Oxidationsfixiermittelformulierung und die Farbstofflösung beim Anwenden im Verhältnis 1:1 gemischt wurden. Hierdurch wurde das graue Haar von der Wurzel bis zur Spitze gleichmäßig in Wellen gelegt und violett gefärbt.

### Ausführungsbeispiel 5

Zweite Teilkomposition des Dauerwellmittels mit Natriumbromat.

Nach üblichem Verfahren wurde die zweite Teilkomposition des Dauerwellmittels als eine 2K-Zusammensetzung, bestehend aus einem Oxidationsfixiermittel und einen Farbstoffpulver, gemäß folgenden Rezepturen zubereitet.

### Oxidationsfixiermittel :

| Inhaltsstoffe | % |
|---|---|
| Natriumbromat | 8,5 |
| Lauryldimethylessigsäurebetain | 1,0 |
| Cetyltrimethylammoniumchlorid | 0,6 |
| Natriumbenzoat | 0,3 |
| Salicylsäure | 0,05 |
| Trinatriumphosphat | 0,27 |
| Phosphorsäure, gereinigtes Wasser | ad 100 |

(mit Phosphorsäure auf pH 6,5 eingestellt)

### Farbstoffpulver :

| Inhaltsstoffe | % |
|---|---|
| (1-Methyl-1-para-methoxyphenyl)-2-(1-methin- | 0,02 |
| 4N-Methylpyridinium) hydrazinchlorid | |
| 3-Amino-7-(dimethylamino)-2-methoxyphenoxazin- | 0,02 |
| 5-iumchlorid | |
| %-Angabe : bezogen auf das Gewicht des Oxidationsfixiermittels | |

Als erste Teilkomposition wurde eine solche wie im Beispiel 3 verwendet.

Graues Haar wurde auf gleicher Weise wie im Beispiel 1 dem Dauerwellbehandlungs- und Färbevorgang unterzogen, außer daß das Oxidationsfixiermittel und das Farbstoffpulver beim Anwenden im Verhältnis 10:1 gemischt wurden. Hierdurch wurde das weiße Haar von der Wurzel bis zur Spitze gleichmäßig in Wellen gelegt und grün gefärbt.

### Ausführungsbeispiel 6

Verfahren zum Färben der dauerwellvorbehandelten Haare.

Die Farbstofflösung mit folgender Zusammensetzung wurde hergestellt :

| Inhaltsstoffe | % |
|---|---|
| (1-Methyl-1-para-methoxyphenyl)-2-(1-methin- | 0,2 |
| 4N-Methylpyridinium) hydrazinchlorid | |
| Hydroxyethylcellulose | 2,5 |
| Triethanolamin, gereinigtes Wasser | ad 100 |

(mit Triethanolamin auf pH 8,0 eingestellt).

Als Oxidationsfixiermittel wurde ein solches wie im Beispiel 5 und als erste Teilkomposition eine solche wie im Beispiel 3 verwendet.

Wie im Beispiel 1 wurde graues Haar mit der ersten Teilkomposition und dem Oxidationsfixiermittel dauerwellbehandelt.

Hierdurch wurde das graue Haar von der Wurzel bis zur Spitze in Wellen gleichmäßig gelegt und braun gefärbt. Danach wurde das weiße Haar mit Farbstofflösung bestrichen, 30 Minuten bei 30°C belassen, mit Wasser gespült, schampuniert und getrocknet. Hierdurch wurde das graue Haar von der Wurzel bis zur Spitze gleichmäßig in Wellen gelegt und gelb gefärbt.

### Gewerbliche Anwendbarkeit

Erfindungsgemäß wird durch Mischung oder Kombination der bisherigen zweiten Teilkomposition des Dauerwellmittels mit bestimmten kationischen Farbstoffen eine zweite Teilkomposition des Dauerwellmittels, die sich durch Stabilität, Färbevermögen und Echtheiten auszeichnet, angeboten. Erfindungsmäßig werden außerdem bei dem der Dauerwellbehandlung nachfolgenden Färben der Haare mit der o. g. Farbstofformulierung hohe Farbausbeute und Echtheiten ohne Beeinflussung des Dauerwelleffektes erhalten.

## Patentansprüche

1. Dauerwellmittel, bestehend aus einem ersten Mittel, dem Reduktionsmittel, und einer zweiten Teilkomposition, dem oxidierenden Fixiermittel, **dadurch gekennzeichnet, dass** die zweite Teilkomposition des Dauerwellmittels einen kationischen Farbstoff enthält, der ein quartäres Stickstoffatom, dessen freies Elektronenpaar delokalisiert sein kann, und eine -X=N-Bindung aufweist, wobei X für ein Stickstoffatom oder eine Gruppe -CHsteht.

2. Dauerwellmittel, nach Anspruch 1, **dadurch gekennzeichnet, dass** in der zweiten Teilkomposition kationische Farbstoffe der Formel (I) eingesetzt sind:
[A-Z=N-B]⁺X⁻
worin
Z Stickstoff oder -CH- bedeutet,
A und B einen aromatischen Benzolring oder Heterozyklus, der je gewünschtenfalls mit einem oder mehreren Halogenatom oder einer oder mehreren NR¹R²- bzw. OR¹-Gruppen substituiert sein kann, darstellt, wobei R¹ und R² gleich oder verschieden sein können und unabhängig voneinander bedeuten jeweils Wasserstoff, C₁₋₈-Alkyl, C₁₋₄-Hydroxyalkyl oder Phenyl, und
X ein Anion ist.

3. Dauerwellmittel nach Anspruch 1, **dadurch gekennzeichnet, dass** die zweite Teilkomposition 0,001 - 3 Gew.-% kationische Farbstoffe enthält.

4. Dauerwellmittel nach Anspruch 1, **dadurch gekennzeichnet, dass** die zweite Teilkomposition als Zweikomponentenmittel, bestehend aus getrennten Formulierungen der Farbstoffe und des Oxidationsfixiermittels, formuliert ist, die beide erst beim Verwenden miteinander gemischt werden.

5. Verfahren zum Dauerwellen und Färben von Haaren, **dadurch gekennzeichnet, dass** das Färben der Haare mit einer Formulierung durchgeführt wird, die einen kationischen Farbstoff, der ein quartäres Stickstoffatom, dessen freies Elektronenpaar delokalisiert sein kann, und eine -X=N-Bindung aufweist, wobei X für ein Stickstoffatom oder eine Gruppe -CH- steht, enthält, wobei der Farbstoff in der zweiten Teilkomposition des Dauerwellmittels enthalten ist.

6. Verfahren nach Anspruch 5, **dadurch gekennzeichnet, dass** die kationischen Farbstoffe der Formel (I) nach Anspruch 2 verwendet werden.

7. Verfahren nach Anspruch 5, **dadurch gekennzeichnet, dass** die Einsatzmenge der kationischen Farbstoffe 0,001 - 3 Gew.-%, bezogen auf die betreffende Komposition, beträgt.

8. Verfahren nach Anspruch 5, **dadurch gekennzeichnet, dass** die verwendete Farbstoffformulierung beim Färben in Form einer wässrigen Lösung bei pH ≥ 5 vorliegt.

9. Verfahren nach Anspruch 8, bei dem die verwendete Farbstoffformulierung eine wässrige Lösung ist, deren Gehalt an Wasser über 60% beträgt.

## Claims

1. Permanent wave composition consisting of a first agent, the reducing agent, and a second part composition, the oxidizing neutralizer, **characterized in that** the second part composition of the permanent wave composition comprises a cationic dye which has a quaternary nitrogen atom whose free electron pair can be delocalized and a -X=N-bond, where X is a nitrogen atom or a group -CH-.

2. Permanent wave composition according to Claim 1, **characterized in that,** in the second part composition, cationic dyes of the formula (I) are used:
[A-Z=N-B]⁺X⁻
in which
Z is nitrogen or -CH-,
A and B are an aromatic benzene ring or heterocycle, which can in each case be substituted if desired by one or more halogen atoms or one or more NR¹R² or OR¹ groups, where R¹ and R² may be identical or different and, independently of one another, are in each case hydrogen, C₁₋₈-alkyl, C₁₋₄-hydroxyalkyl or phenyl, and
X is an anion.

3. Permanent wave composition according to Claim 1, **characterized in that** the second part composition comprises 0.001-3% by weight of cationic dyes.

4. Permanent wave composition according to Claim 1, **characterized in that** the second part composition is formulated as two-component composition, consisting of separate formulations of the dyes and of the oxidation neutralizer, which are both only mixed together upon use.

5. Method for the permanent waving and dyeing of hair, **characterized in that** the dyeing of the hair is carried out using a formulation which comprises a cationic dye which has a quaternary nitrogen atom whose free electron pair can be delocalized and a -X=N bond, where X is a nitrogen atom or a group -CH-, where the dye is present in the second part composition of the permanent wave composition.

6. Method according to Claim 5, **characterized in that** the cationic dyes of the formula (I) according to Claim 2 are used.

7. Method according to Claim 5, **characterized in that** the use amount of the cationic dyes is 0.001-3% by weight, based on the composition in question.

8. Method according to Claim 5, **characterized in that** the dye formulation used for the dyeing is present in the form of an aqueous solution at pH ≥ 5.

9. Method according to Claim 8, in which the dye formulation used is an aqueous solution whose content of water is above 60%.

## Revendications

1. Agent pour permanente comprenant un premier agent, l'agent réducteur, et une deuxième composition partielle, l'agent de fixation oxydant, **caractérisé en ce que** la deuxième composition partielle de l'agent pour permanente contient un colorant cationique qui possède un atome d'azote quaternaire dont la paire d'électrons libre peut être délocalisée et une liaison -X=N- où X représente un atome d'azote ou un groupe -CH-.

2. Agent pour permanente selon la revendication 1, **caractérisé en ce que** dans la deuxième composition partielle sont utilisés des colorants cationiques répondant à la formule (I) :
[A-Z=N-B]⁺X⁻
où
Z désigne un atome d'azote ou un groupe -CH-,
A et B représentent un cycle benzénique ou un hétérocycle aromatique qui, dans chaque cas, si on le souhaite, peut être substitué par un ou plusieurs atomes d'halogène ou par un ou plusieurs groupes NR¹R² ou OR¹, où R¹ et R² peuvent être identiques ou différents et représentent chacun indépendamment l'un de l'autre un atome d'hydrogène, un groupe alkyle en C₁₋₈, un groupe hydroxyalkyle en C₁₋₄ ou phényle, et
X est un anion.

3. Agent pour permanente selon la revendication 1, **caractérisé en ce que** la deuxième composition partielle contient de 0,001 à 3 % en poids de colorants cationiques.

4. Agent pour permanente selon la revendication 1, **caractérisé en ce que** la deuxième composition partielle est formulée sous la forme d'un agent à deux composants comprenant des formulations séparées des colorants et de l'agent de fixation d'oxydation, toutes deux n'étant mélangées entre elles que lors de l'utilisation.

5. Procédé pour permanente et coloration de cheveux, **caractérisé en ce que** la coloration de cheveux est effectuée avec une formulation qui contient un colorant cationique comprenant un atome d'azote quaternaire dont la paire d'électrons libres peut être délocalisée et une liaison -X=N- où X représente un atome d'azote ou un groupe -CH-, le colorant étant contenu dans la deuxième composition partielle de l'agent pour permanente.

6. Procédé selon la revendication 5, **caractérisé en ce que** les colorants cationiques répondant à la formule (I) sont utilisés selon la revendication 2.

7. Procédé selon la revendication 5, **caractérisé en ce que** la quantité d'addition des colorants cationiques est de 0,001 à 3 % en poids sur la base de ladite composition.

8. Procédé selon la revendication 5, **caractérisé en ce que** la formulation de colorants utilisés lors de la coloration est présente sous la forme d'une solution aqueuse ayant un pH ≥ 5.

9. Procédé selon la revendication 8 par lequel la formulation de colorants utilisée est une solution aqueuse dont la teneur en eau est supérieure à 60 %.
